# EUROPEAN PATENT APPLICATION

(11) **EP 1 884 250 A1**
(43) Date of publication of application: **06.02.2008**
(21) Application number: 06398011.4
(22) Date of filing: 03.08.2006
(51) Int. Cl.: A61L 2/20, A61L 101/02

(54) **Low temperature steriliser using hydrogen peroxide vapour**

(71) Applicant: Goncalvez, Helder Da Costa, 5460-909 Boticas (PT)
(72) Inventor: Goncalvez, Helder Da Costa, 5460-909 Boticas (PT)

(57) **Abstract**

The machine sterilizes with hydrogen peroxide vapour produced in a dedicated vaporizer made of stainless steel and then injected into the sterilization chamber (also made in stainless steel).
The hydrogen peroxide is withdrawn from the chamber passing through a burning process with high tension in the burner.
The vapour is withdrawn from the material by ventilation composed by an air entry and vacuum in the chamber with its following burning.
This equipment is able to sterilize rigid and length flexible tubes. The flexible ones can have 10 m length and a 1 mm diameter, and the rigid tubes can have the total size of the chamber with a 1 mm diameter.

## Description

### Development basis:

This process study is based on sterilization system by formaldehyde, where in this cycle it was been put a dose of approximately 80 cm³ of 30% formaldehyde in a generator heated by steam in order to vaporize the formaldehyde into a chamber in vacuum with 760 mHg.

In our method, we have altered the way to transform in hydrogen peroxide steam being generated by an electrical boiler with pressure and temperature control. The boiler has an autonomous cooling system.
We have used the hydrogen peroxide for being a sterilant agent whom decomposed in plasma is also a sterilant agent.

### Tests and results:

We have tested the sterilant effect of the hydrogen peroxide steam in a very small chamber, heating the chamber and injecting with a syringe specified quantities of hydrogen peroxide in order to evaluate the biological kill.

In theses preliminary tests we have used bacillus stearothermophilus 10⁶ in a stainless steel carrier. We have detected that it is possible to sterilize the small stainless steel carriers with bacillus stearothermophilus. Naturally, we have used incubators and tests of market except the tests which have been produced for the preliminary tests. These tests have been asked to the manufacturer with different populations 10³, 10⁴, 10⁵, 10⁶ and different types of carriers.
In these preliminary tests we have choose the testes in a stainless steel carrier tests because they present too much fails and are the more difficult to sterilize.
Because of the obtained results were appointing with clarity the method possible efficiency, we resolve to go away with the mechanical tests, in order to obtain the same results , but now with a 62 litres chamber.

### Mechanical study:

The mechanical study has been started in order to burn the hydrogen peroxide vapour, we have used, as base, a neon gas burner. We made a metallic item with two applied electrodes and we also used a various transformers for high tension burning of the hydrogen peroxide steam. The burning has been tested. We used and changed the hydrogen peroxide quantity at the enter of the burner and measured the hydrogen peroxide at the exit of the burner. We have used a hydrogen peroxide sensor with capacity to read 0,1 PPM. We have concluded that the burner could be done based in stainless steel visor with two electrodes, because the read results were less than 0,1 PPM.

We have studied the hydrogen peroxide steam generator using, as initial study a 60 mm diameter cylinder with a resistance in the outside. We have used a PT 100 probe to control the steam production and concluded that its efficiency could be increased if we put a transducer to command the pressure of the vapour produced in the generator. For security reasons we also put a controlled ventilation system in this element.

*Electrical test and position of the sensors:*

### 1) Electrical test:

We have studied the enters and exits into the hydrogen peroxide vapour circuit with a 62 litres chamber with two doors and used clamp connections. The circuit has been used to put vapour in a point of the chamber, with the exhaust in the opposite side. In order to obtain a burning we need vacuum values less than 40 mBar. We choose a vacuum pump able to do 1 mBar, and connected the vacuum pump to the chamber with a pneumatic valve. We have studied the chamber configuration in order to have two hinged doors with manual lock system. For the sealing of the doors, we have used a silicone cylinder-joint.
We have repeated the tests made with a micro chamber, but now with a bigger chamber, and we concluded that it was necessary to warm the chamber and the doors.

Because it was necessary to warm the chamber and the doors, we studied a conductor type resistance that we involved in the chamber outside. We put a PT 100 probe for the temperature control in the lateral side near the bottom of the chamber in order to obtain the coolest point of the chamber. Then, we have covered the resistance with mineral wool and measured the temperature stability. We have concluded that it is necessary to warm the doors. We have made plane resistances plate type and we have applied PT 100 probes in the doors in order to control. We also have isolated the doors with mineral wool and obtained a chamber and doors temperature homogeneity.

### 2) Position of the sensors:

Once the chamber has been constructed, we have proceeded to the position of command system of the machine, at the temperature and pressure level.
As we have mentioned, we put a probe in the autoclave body and a probe in each door. We have put a probe in the burner, because it seems to be a vital point in the cycle, and very important on the hydrogen peroxide vapour burning.
We have put a probe in the hydrogen peroxide tank, because the hydrogen peroxide is more stable with temperatures less than 30°C. We have put an automatic system for the cooling of the tank. We have put a temperature probe in the hydrogen peroxide steam generator for security and ventilation system.
We have put two micro switches and two magnetic locks, in order the doors give lock indication. These ones assure that the doors are closed during the cycle. In the chamber, we have put a pressure transducer that sends pressure values to the command. The software does the management of the needed values for the sterilization.

### Hydrogen peroxide steam generator:

The hydrogen peroxide steam generator is made in stainless steel with a resistance controlled by a temperature probe and a pressure transducer. It has an electrical valve for the hydrogen peroxide alimentation at the entrance and an electrical valve at the exit of the chamber. The alimentation of the hydrogen peroxide is assured by a peristaltic pump which abstracts the hydrogen peroxide from a tank and put it in the hydrogen peroxide steam generator. We put a ventilator to cool the vaporizer.

The tank of the hydrogen peroxide is made in stainless steel with a capacity of 800 ml even if 2/3 of its total volume is used. It has, as main characteristic, a level system by magnetic rides and an autonomous system of control of the temperature with Peltier plates cooling. It has an electrical valve connected to a respirator which allows the entrance of the hydrogen peroxide into the tank and the exit to the hydrogen peroxide vapour generator. All its surface is thermally isolated and its temperature is less than 30°C.

### Hydrogen peroxide steam Burner:

The hydrogen peroxide steam burner is made in stainless steel with two electrodes prepared for the effect. It has two glasses which are used as a visor and a PT 100 probe which indicates the correct operation. Its entrance and exit are made by rapid connections also in stainless steel. The operation temperature is higher than 100°C.

### Vacuum pump:

It is able to execute a vacuum of 1 mBar, it has a filter which separates the oil vapour and has an odour filter.

### Valves:

The drain valve is made of stainless steel and has a pneumatic actuation with a clamp connection.
The air-entraining valves, of hydrogen peroxide entrance and exit are made in stainless steel with a 24V DC coil acting.

### Bottle support:

This support has an automatic punch with a pneumatic system of drive which punches the bottle and abstracts the sterilant agent into the tank. This operation is totally automatic commanded by the software management system.
This support has a bar code reader which assures that the right product is put into the sterilizer. It does not work with another type of product and does not allow the utilization of the same bottle of sterilant agent more than one time, because all the supplies, the date of manufacturing and all the characteristics about the bar code are registered in the computer system.

### Mechanical test:

After constructing the chamber and the application of the doors, we have made sealing tests of the chamber putting the camber in vacuum during a period of time and analyzing the difference between the initial and final vacuum values. We have created a mechanical chamber support structure which allows the application of removable panels.

We have distributed the different components of the machine this way: being in the loading side we have the supply system and the alimentation of the hydrogen peroxide and the hydrogen peroxide steam generator supplier on the right side.
On the lower level, we have put a transformer, a vacuum pump and the burner.
On the upper level, we have put various types of electrical commands, air-entraining filter and a transducer. The entrance of the hydrogen peroxide vapour has been put in the upper level. The control panel has been put in the loading side.

The machine structure is totally made in stainless steel, and totally dismountable. The panels' application of the cover is rapid to fit in order to facilitate the assistance. All the panels are made in stainless steel.

### Vapour flow:

The hydrogen peroxide generator has been made for the vaporization of this one and injects it into the machine in order the vapour enters in the chamber without condensing. During the sterilization cycle, the chamber keeps the hydrogen peroxide vapour in a gaseous state, at the defined value of hydrogen peroxide. The vaporization in the chamber occurs when the vacuum value is between 1 and 40 mBA, supported by the temperature inside the chamber and the doors. The injection of the hydrogen peroxide is dosed at the chamber entry into the inside of the chamber; it has got a calibrated injector with the steam generator to maintain the hydrogen peroxide vaporized.

### Biological tests:

We have made biological tests with various types of lumens where we had introduced bacillus stearothermophilus spores in stainless steel carriers created for this effect, with populations of 10³, 10⁴, 10⁵ and 10⁶. Various types of tests and types of cycles were made for the sterilization of the various types of tests.
We have increased the difficulty degree of the biological material using higher population tests adjusting the cycle. In the cycle, established as pattern, we got tests sterilization with a population of 10⁶ in the long cycle and 10⁵ in the fast cycle. We noticed that the autoclaves manufacturers who sterilize by plasma of hydrogen peroxide use lumens that they had established as pattern. This kind of lumens has been used in our tests with various types of population, in the stainless steel carriers with bacillus stearothermophilus.

### Burning tests:

We have made tests manually injecting hydrogen peroxide vapour into the burner.
The burner was made in order the hydrogen peroxide vapour is eliminated before being sended to the atmosphere. We managed this objective with a chamber in stainless steel where we put two ignition plugs and we have applied a high tension. We also have in the burner a probe that assures the correct operation during the burning period, which gives, in case of burner failure, indications to the PLC which blocks the cycle and informs the operator.

The tests are based in the injection of hydrogen peroxide vapour in different concentrations with the measurement of the residual hydrogen peroxide vapour values at the vacuum pump exit. The burner has been physically studied with the purpose that we have values of hydrogen peroxide vapour less than 1 PPM at the exit of the vacuum pump. The burner volume and the electrodes distance are directly related with the size of the sterilization chamber and the quantity of hydrogen peroxide vapour put into the sterilizer chamber. All the final tests values are less than 1 PPM.

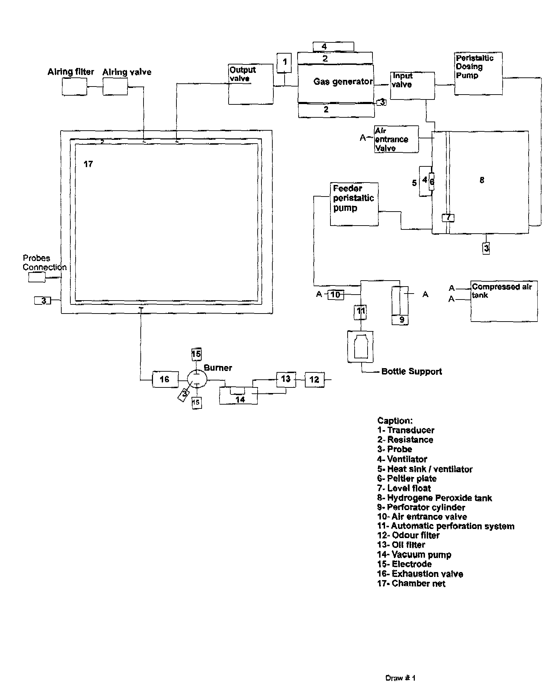

## Claims

1. The main innovation of this low temperature sterilizer is to sterilize with hydrogen peroxide vapour with the cleaning of the sterilized material during the final aeration phase of the cycle.

2. To reach the vaporization, we have developed an innovation which consists in a generator supply system of the hydrogen peroxide steam, where we have dosed the quantity of hydrogen peroxide before putting it into the generator. The hydrogen peroxide steam generator control is made by the temperature and pressure.

3. Another innovation is that this hydrogen peroxide vapour generator has a cooler for the stabilization of the temperatures inside and outside the hydrogen peroxide steam generator.
All this assembly of entrance of hydrogen peroxide vapour into the chamber and into the hydrogen peroxide steam generator is composed by a sealed block where the connections are available.

4. Another innovation is the hydrogen peroxide burner. In other manufacturers it is a plasma generator which put the plasma into the chamber.
In our machine, the burner's purpose is only the burning of the hydrogen peroxide vapour; it means its chemical alteration. This burner is controlled by a temperature probe and the burning is maintained by a high tension bunch at a vacuum value studied for the effect. At the burner exit, we have water vapour and oxygen.

5. In our system we have another innovation, which consists in the automatic detection of the bottles with automatic pneumatic punching. Automatic pumping of the product to the tank. This system is a closed block.

6. Another innovation is the hydrogen peroxide tank where we have put Peltier plates for the cooling. We have fit temperature probes in order to control the temperature. It has a level control system by magnetic rides, which is an exclusive development made by ourselves for this hydrogen peroxide tank and is a part of the tank block.

7. The developed cycle is also an innovation because of the steps is has got. It starts with a vacuum, (vacuum pump maximum value is of 1 mBar), then a air dragging is made in the chamber in order to abstract any humidity. After, the hydrogen peroxide vapour is injected into the chamber dosed with a studied pressure value in order to have sterilization with hydrogen peroxide vapour and in order the vapour reaches all the points of the load. After the end of the sterilization step, there is a hydrogen peroxide vapour discharge into the burner chamber, with controlled flow to allow the burning. During this period, the hydrogen peroxide vapour is dragged from the sterilization chamber and burned in the burner until the sterilization chamber has got less than 1 PPM of hydrogen peroxide in the materials.
